# FASCICULE DE BREVET EUROPEEN

(11) **EP 3 261 633 B1**
(45) Date de publication et mention de la délivrance du brevet: **19.09.2018**
(21) Numéro de dépôt: 16705554.0
(22) Date de dépôt: 22.02.2016
(51) Int. Cl.: A61K 31/20, A61K 31/352, A61K 31/522, A61Q 7/00, A61P 17/14, A61K 8/49, A61K 8/36, A61Q 5/02

(54) **COMPOSITION COSMÉTIQUE OU DERMATOLOGIQUE POUR LE TRAITEMENT DE L'ALOPÉCIE**
KOSMETISCHE ODER DERMATOLOGISCHE ZUSAMMENSETZUNG ZUR BEHANDLUNG VON ALOPEZIE
COSMETIC OR DERMATOLOGICAL COMPOSITION FOR TREATING ALOPECIA

(30) Priorité: 24.02.2015 FR 1551574
(43) Date de publication de la demande: 03.01.2018
(73) Titulaire: Galephar M/F, 6900 Marche-en-Famenne (BE)
(72) Inventeur: BAUDIER, Philippe, 20000 Casablanca (MA)
(74) Mandataire: Hirsch & Associés
(86) Numéro de dépôt international: PCT/EP2016/053672
(87) Numéro de publication internationale: WO 2016/135098

(56) Documents cités:
- EP-A1- 1 927 378
- CN-B- 101 869 541
- US-A1- 2003 152 588
- MARÍA JESÚS CEJUDO-BASTANTE ET AL: "Combined Effects of Prefermentative Skin Maceration and Oxygen Addition of Must on Color-Related Phenolics, Volatile Composition, and Sensory Characteristics of Airén White Wine", JOURNAL OF AGRICULTURAL AND FOOD CHEMISTRY, vol. 59, no. 22, 23 novembre 2011 (2011-11-23), pages 12171-12182, XP055236347, US ISSN: 0021-8561, DOI: 10.1021/jf202679y
- Anonymous: "Quercetin - Wikipedia, the free encyclopedia", , 18 décembre 2014 (2014-12-18), XP055236371, Extrait de l'Internet: URL:https://web.archive.org/web/2014121808 1428/http://en.wikipedia.org/wiki/Querceti n [extrait le 2015-12-15]
- Anonymous: "Lauric acid - Wikipedia, the free encyclopedia", , 27 octobre 2014 (2014-10-27), XP055236366, Extrait de l'Internet: URL:https://web.archive.org/web/2014102717 4654/http://en.wikipedia.org/wiki/Lauric_a cid [extrait le 2015-12-15]
- TONGYU CAO WIKRAMANAYAKE ET AL: "Prevention and treatment of alopecia areata with quercetin in the C3H/HeJ mouse model", CELL STRESS AND CHAPERONES ; A COMPREHENSIVE JOURNAL OF STRESS BIOLOGY AND MEDICINE, SPRINGER NETHERLANDS, DORDRECHT, vol. 17, no. 2, 1 novembre 2011 (2011-11-01), pages 267-274, XP035013316, ISSN: 1466-1268, DOI: 10.1007/S12192-011-0305-3
- JOHN MCCOY ET AL: "BOTANICAL EXTRACTS FOR THE TREATMENT OF ANDROGENETIC ALOPECIA", INTERNATIONAL JOURNAL OF LIFE SCIENCE AND PHARMA RESEARCH, vol. 2, no. 4, 1 octobre 2012 (2012-10-01) , pages P-31, XP055236176, ISSN: 2250-0480, DOI: www.ijlpr.com/admin/php/uploads/154_pdf.pd f
- BUSSOLETTI C ET AL: "Use of a cosmetic caffeine lotion in the treatment of male androgenetic alopecia", JOURNAL OF APPLIED COSMETOLOGY 2011 INTERNATIONAL EDIEMME ITA, vol. 29, no. 4, octobre 2011 (2011-10), pages 167-180, XP055236382, ISSN: 0392-8543
- BANSAL MANISH ET AL: "Role of caffeine in the management of androgenetic alopecia", INTERNATIONAL JOURNAL OF TRICHOLOGY, MEDKNOW PUBL, INDIA , vol. 4, no. 3 1 janvier 2012 (2012-01-01), pages 185-186, XP008178458, ISSN: 0974-9241, DOI: 10.4103/0974-7753.100096 Extrait de l'Internet: URL:http://www.ncbi.nlm.nih.gov/pmc/articl es/PMC3500065/?report=printable [extrait le 2012-08-24]

## Description

### Domaine technique

La présente invention concerne une composition à visée dermatologique et cosmétique pour une utilisation en tant que médicament pour le traitement de l'alopécie et plus particulièrement de l'alopécie androgénique, de l'alopécie aérata et de l'alopécie induite par la chimiothérapie ainsi que son utilisation en cosmétique pour la stimulation de la croissance capillaire et comme anti-chute de cheveux .

La composition de la présente invention est obtenue par l'association synergique de la quercétine avec de l'acide laurique.

### Arrière-plan technologique

### Rappel sur le cycle pilaire et l'alopécie :

Le cycle pilaire comprend les 3 phases suivantes :
Phase anagène : cette première phase, est la phase de croissance du cheveu 80% à 85 %des cheveux sont en phase anagène chez l'homme et 85 à 90 % chez la femme. Chaque cheveu pousse en moyenne entre 1 à 1,5 cm par mois. La durée de cette phase varie de 2 à 4 ans chez l'homme et de 4 à 6 ans chez la femme.
Phase catagène : cette seconde phase est marquée par un arrêt de la croissance du cheveu, c'est une phase transitoire courte de 3 semaines qui concerne peu de cheveux 25 à 30 par jour soit 0 à 2 % des cheveux.
Phase télogène : cette dernière phase est celle de la chute des cheveux, le bulbe se kératinise, le follicule pileux régresse de plus de la moitié de sa longueur initiale et tombera après 3 mois, poussé par le nouveau cheveu en cours de formation.

Chaque follicule pileux a un développement indépendant, un follicule pourra être en phase anagène et son voisin en phase télogène.

Les deux types d'alopécies les plus répandus sont l'alopécie androgénique (AAG) et l'alopécie aerata. On peut aussi citer l'alopécie induite par la chimiothérapie.

L'AAG est un processus de chute anormale des cheveux qui touche 40à 50 % des hommes avant 50 ans et 25 à 30 % des femmes, après 50 ans 60 à 80 % des hommes et 40 à 50 % des femmes .

L'AAG est la conséquence d'un excès de conversion de la testostérone en dihydrotestostérone (DHT) au niveau du follicule pileux sous l'action d'une enzyme, la 5-alpha réductase. La DHT va pénétrer dans le noyau du follicule pileux et inhiber l'adényl cyclase, diminuant le taux d'AMP cyclique, ce qui provoque une diminution de la synthèse protidique. La conséquence est un raccourcissement de la phase anagène et du cycle pilaire (Hoffmann R, Hormonal interaction and hair growth ; Ann. Dermatol. Venereol, 2002, May, 129 p 787-792).

Les gènes responsables de l'AAG n'ont pas encore été tous identifiés. Le polymorphisme du gène récepteur aux androgènes a été identifié par Axel Hilmer (Axel Hilmer et al. Nat Genet Nov., 40 (11) 1279- 1281), comme déterminant dans l'alopécie androgénique.

L'AAG peut être traitée par plusieurs médicaments. Le Minoxidil, prescrit dans les années 1960 dans le traitement de l'hypertension artérielle, provoquait une hypertrichose chez plus de 70 % des patients. L'utilisation per os est contre indiquée pour ses effets secondaires cardiovasculaires. En application topique les effets indésirables sont principalement de types cutanés : irritation locale, desquamation, érythème, dermite ; ainsi que des affections du système nerveux telles que les céphalées. Le mode d'action du Minoxidil reste encore hypothétique, dépourvu d'effet anti-androgène il possède un effet vasodilatateur, un effet mitogénétique et prolonge la durée de vie des kératinocytes. Le Finastéride qui est un inhibiteur de la 5-alpha réductase, bloque la transformation de la testostérone en dihydrotestostérone (DHT). Ce produit a obtenu une AMM en 1998 dans le traitement des stades peu évolués de l'alopécie androgénique masculine. Le Dutastéride, également un inhibiteur de la 5-alpha réductase, est commercialisé dans le traitement de l'hypertrophie bénigne de la prostate. Ce produit a été testé dans le traitement de l'alopécie de stade II, III ou IV. L'acétate de cyprotérone, un dérivé de l'hydroxyprogestérone est considéré comme l'anti-androgène de référence. Ce produit est commercialisé dans le traitement du cancer de la prostate, de l'hirsutisme chez la femme en association avec un oestrogène. Son action anti-séborrhéique est rapide, mais l'action anti-chute semble plus lente et moins constante. L'acétate de cyprotérone est indiquée hors AMM dans l'alopécie androgénétique féminine (AAGF) associée à une hyper-séborrhée. La Spironolactone est un antagoniste de l'aldostérone, ce qui lui confère une action anti-hypertensive et diurétique. Elle possède également une action antagoniste des androgènes, ainsi qu'une action d'inhibition de la synthèse de la testostérone. La spironolactone a été utilisée hors AMM dans le traitement de l'AAGF et a montré de bons résultats anti-chute, mais rarement une repousse des cheveux. Le document "Cell Stress and Chaperones (2012) 17:267-274" ainsi que le document EP 1 927 378 décrivent le traitement de l'alopécie et de la chute de cheveux par la quercétine. Le document "International Journal of Life Science and Pharma Research, Vol.2, Issue 4, 2012, pages 31-38" décrit les effets bénéfiques de l'acide laurique dans le traitement de l'alopécie androgénique.

### Résumé de l'invention

La présente invention a précisément pour but de proposer une nouvelle composition à visée dermatologique et cosmétique pour une utilisation en tant que médicament pour le traitement de l'alopécie et plus particulièrement de l'alopécie androgénique, de l'alopécie aérata et de l'alopécie induite par la chimiothérapie dans le but d'améliorer les performances des produits actuellement commercialisés et/ou de diminuer leurs effets secondaires.

L'inventeur de la présente invention a constaté de façon surprenante que l'association de la quercétine avec de l'acide laurique est capable de potentialiser la pousse des cheveux.

La présente invention concerne donc une composition comprenant au moins de la quercétine et au moins de l'acide laurique.

Cette association synergique selon l'invention, permet d'obtenir des résultats sur la repousse des cheveux au moins équivalents à ceux du Minoxidil tout en évitant ses effets secondaires cutanés et son défaut de rémanence lors de l'arrêt du traitement.

L'acide laurique également appelé acide n-dodécanoïque, est un acide gras saturé à 12 atomes de carbone, de formule semi-développée CH3-(CH2)10-COOH. On le trouve notamment dans l'huile de coco et dans l'huile de palmiste, deux huiles alimentaires particulièrement riches en acide laurique et en acide myristique.

L'acide laurique est le principal acide gras de l'huile de coprah (huile du cocotier), dont il constitue environ 50 % des acides gras. On le trouve également en faible quantité dans le lait de vache ainsi que dans le lait maternel humain. Ses propriétés antimicrobiennes seraient très importantes pour les nourrissons puisque leur système immunitaire n'est pas complètement développé.

La Quercétine ou quercétol est un flavonoïde de type flavonol présent chez les plantes comme métabolite secondaire. Le quercétol est le plus actif des flavonoïdes et de nombreuses plantes médicinales doivent leur efficacité à leur fort taux en quercétol. Les études in vitro et in vivo ont montré que c'était un excellent anti-oxydant.

La caféine est un actif optionnel dans la présente composition. Cet actif est un stimulant du système nerveux central et du métabolisme ; il est utilisé pour réduire la fatigue physique et restaurer la vigilance quand une faiblesse ou une somnolence inhabituelle se produit. La caféine stimule le système nerveux central au niveau du cerveau, il en résulte une vigilance accrue, un flot de pensées plus claires et rapides, une augmentation de la concentration et une coordination générale du corps améliorée.

D'autres caractéristiques et avantages de la présente invention apparaîtront à la lecture de la description détaillée qui suit et du mode de réalisation préféré de l'invention, donnée à titre d'exemple.

### Description détaillée

La Demanderesse a trouvé, de façon surprenante et inattendue, que la combinaison synergique de la quercétine avec de l'acide laurique permet le traitement de l'alopécie et plus particulièrement de l'alopécie androgénique, de l'alopécie aérata et de l'alopécie induite par la chimiothérapie ainsi que son utilisation en cosmétique pour la stimulation de la croissance capillaire et comme anti-chute de cheveux.

Dans un mode de réalisation particulier, la quercétine représente de 0,01 à 0,5 % en poids, de préférence 0,1 à 0,5 % en poids, de préférence de 0,2% en poids par rapport au poids total de la composition.

Dans un mode de réalisation particulier, l'acide laurique représente de 0,05 à 2,5% en poids, de préférence de 0,1 à 2% en poids, de préférence de 0,5 à 1% en poids par rapport au poids total de la composition.

Dans un mode de réalisation préférée, le rapport quercétine/ acide laurique est de 1 :5.

Dans un mode de réalisation particulier, la composition comprend en outre de la caféine qui représente de 0,01 à 1% en poids, de préférence de 0,1 à 1% en poids, de préférence de 0,3 à 0,5% en poids par rapport au poids total de la composition.

Dans un mode de réalisation particulier, la composition selon l'invention est utilisé pour la fabrication d'un traitement, en particulier cosmétique ou dermatologique ayant un effet synergique sur la repousse des cheveux et l'action anti-chute capillaire de préférence dans une proportion de quercétine /acide laurique de 1 :5. Les effets décrits sont également observés lorsque la composition synergique comprend une proportion en poids de quercétine /acide laurique comprise entre 1 :3 et 1 :7.

Selon un mode de réalisation particulier, la composition selon l'invention comprend en outre un véhicule cosmétiquement ou pharmaceutiquement acceptable.

Par « véhicule cosmétiquement ou pharmaceutiquement acceptable », on entend toute substance adaptée à une utilisation en contact avec la peau, et en particulier les cellules de l'épiderme, sans toxicité, incompatibilité, instabilité, irritation, réponse allergique ou similaire, avec un rapport bénéfice / risque raisonnable.

Les compositions selon l'invention peuvent se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, notamment de consistance liquide ou semi-liquide, du type HIE, E/H ou multiple; d'une suspension ou émulsion de consistance molle de type crème (H/E) ou (E/H); d'un gel aqueux ou anhydre, ou de toute autre forme cosmétique ou pharmaceutique.

La forme de la composition cosmétique ou pharmaceutique de l'invention n'est pas particulièrement limitée, et la composition cosmétique ou pharmaceutique de l'invention peut être dans une forme quelconque, telle qu'un liquide, une pâte ou un gel. La composition cosmétique ou pharmaceutique de l'invention est spécifiquement de préférence formulée en une composition cosmétique ou pharmaceutique pour la peau et/ou les cheveux et des exemples de celle-ci comprennent des lotions, des crèmes, des émulsions, ou des sérums.

La composition selon l'invention peut être aqueuse. Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale. La composition est de préférence une composition aqueuse et comprend alors de l'eau à une concentration de préférence comprise entre 5 et 98% en poids, notamment 20 et 95% en poids, mieux 50 et 95% en poids, par rapport au poids total de la composition.

Ladite phase aqueuse peut comprendre en outre un ou plusieurs solvants organiques tels qu'un alcool en notamment l'éthanol, l'isopropanol, le tert-butanol, le n-butanol, des polyols comme la glycérine, le propylène glycol, le butylène glycol, l'isoprène glycol, le polyéthylène glycol, macrogol et des éthers de polyol.

Lorsque la composition selon l'invention se présente sous la forme d'une émulsion, elle peut éventuellement comprendre en outre un tensioactif, de préférence en une quantité de 0,01 à 30 % en poids par rapport au poids total de la composition. La composition selon l'invention peut également comprendre au moins un co-émulsionnant qui peut être choisi parmi le monostéarate de sorbitan éthoxylé, le monostérate de sorbitan, le monostérate de glycérol, des alcools gras tels que l'alcool stéarylique ou l'alcool cétylique, ou des esters d'acides gras et de polyols tels que le stéarate de glycéryle.

La composition selon l'invention peut également comprendre une phase grasse, notamment constituée de corps gras liquides à 25°C, tels que des huiles d'origine animale, végétale, minérale ou synthétique, volatiles ou non; de corps gras solides à 25°C tels que des cires d'origine animale, végétale, minérale ou synthétique; de corps gras pâteux; de gommes; de leurs mélanges.

Les huiles volatiles sont généralement des huiles ayant, à 25°C, une tension de vapeur saturante au moins égale à 0,5 millibar (soit 50 Pa).

Parmi les constituants de la phase grasse, on peut citer :
- les silicones volatiles cycliques ayant de 3 à 8 atomes de silicium, de préférence de 4 à 6.
- les cyclocopolymères du type diméthylsiloxane/méthylalkylsiloxane,
- les silicones volatiles linéaires ayant de 2 à 9 atomes de silicium.
- les huiles volatiles hydrocarbonées, telles que les isoparaffines et notamment l'isododécane et des huiles fluorées.
- les polyalkyl(C1-C20) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes, de carthame ou d'avocat; les huiles de poisson (squalane), le tricaprocaprylate de glycérol, ou les huiles végétales ou animales de formule dans laquelle représente le reste d'un acide gras supérieur comportant de 7 à 19 atomes de carbone et R2 représente une chaîne hydrocarbonée ramifiée contenant de 3 à atomes de carbone, par exemple, l'huile de Purcellin ; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales ; des esters d'acides gras ; des alcools ; des acétylglycérides ; des 10 octanoates, décanoates ou ricinoléates d'alcools ou de polyalcools ; des triglycérides d'acides gras (comme par exemple Neobee M5); des glycérides ;
- les huiles fluorées et perfluorées ;
- les gommes de silicones ;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre ; les huiles hydrogénées concrètes à 25°C, les ozokérites, les esters gras et les glycérides concrets à 25 °C ; les cires de polyéthylène et les cires obtenues par synthèse de Fischer-Tropsch ; des huiles hydrogénées concrètes à 25 °C ; des lanolines ; des esters gras concrets à 25°C ; les cires de silicone ; les cires fluorées.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques ou pharmaceutiques hydrophiles ou lipophiles, les conservateurs, les épaississants, les vitamines, les régulateurs de pH, les antioxydants, les solvants, les parfums, les charges, les pigments, les nacres, les filtres UV, les absorbeurs d'odeur et les colorants. Ces adjuvants, selon leur nature, peuvent être introduits dans la phase grasse, dans la phase aqueuse et/ou dans des sphérules lipidiques.

La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

La présente invention a également pour objet une composition sous forme de lotion et/ou de shampooing.

La présente invention a également pour objet une composition topique dermatologique et/ou cosmétique comprenant au moins de la quercétine avec au moins de l'acide laurique pour son utilisation pour la stimulation de la croissance capillaire et comme anti- chute de cheveux. Ladite composition peut également être utilisée comme médicament dans le traitement de l'alopécie, en particulier l'alopécie androgénique, l'alopécie aerata ou l'alopécie induite par chimiothérapie.

La méthode de traitement cosmétique non-thérapeutique de l'invention peut être mis en oeuvre notamment en appliquant les compositions cosmétiques telles que définies ci-dessus, selon la technique d'utilisation habituelle de la composition. Par exemple : application de lotion ou de shampooing sur les cheveux ou le cuir chevelu.

Bien entendu, la présente invention n'est pas limitée aux exemples et au mode de réalisation décrits et représentés, mais elle est susceptible de nombreuses variantes accessibles à l'homme de l'art.

### Exemples :

Les exemples et compositions suivants illustrent l'invention. Dans les compositions les proportions indiquées sont des pourcentages en poids.

Evaluation de l'effet synergique sur la repousse des cheveux et l'action anti-chute capillaire de l'association quercétine avec l'acide laurique :
Les 4 formulations suivantes ont été testées en clinique sous contrôle médical

### Formulation A : lotion capillaire

| Principes actifs : | |
|---|---|
| Quercétine : | 200 mg |
| Acide laurique : | 1 g |

| Excipients : | |
|---|---|
| Propylène glycol | 6 g |
| Alcool éthylique à 96° QSP | 100 g |

### Formulation B

| Principes actifs : | |
|---|---|
| Quercétine : | 200 mg |

| Excipients : | |
|---|---|
| Propylène glycol | 6 g |
| Alcool éthylique à 96° QSP | 100 g |

### Formulation C

| Principes actifs : | |
|---|---|
| Acide laurique : | 1 g |

| Excipients : | |
|---|---|
| Propylène glycol | 6 g |
| Alcool éthylique à 96° QSP | 100 g |

### Formulation D

| Principes actifs : | |
|---|---|
| Minoxidil | 2 g |

| Excipients : | |
|---|---|
| Propylène glycol | 6 g |
| Alcool éthylique à 96° QSP | 100 g |

Pour réaliser cette étude on utilise la méthode classique du phototrichogramme qui est une méthode macrophotographique standardisée.

L'étude consiste à appliquer 1 ml/jour, les formulations, pendant une période de 3 mois sur 40 volontaires (divisés en 4 groupes de 10) âgés de 30 à 65 ans, atteints d'une alopécie légère à modérée (classe II à III selon Hamilton), pour chaque volontaire , un site d'une surface de 1 cm2 est délimité dans la région temporale droite .
Le 1^{er} groupe de 10 volontaires est traité par la formulation A: quercétine + acide laurique
Le 2^{ème} groupe de 10 volontaires par la formulation B : quercétine
Le 3^{ème} groupe de 10 volontaires par la formulation C : acide laurique
Le 4^{ème} groupe de 10 volontaires par la formulation D : minoxidil

Les résultats de cette étude clinique sont mentionnés dans le tableau suivant :

### Modification moyenne du nombre de cheveux :

| | 1^{er} Groupe | 2^{ème} Groupe | 3^{ème} Groupe | 4^{ème} Groupe |
|---|---|---|---|---|
| 1er mois de traitement | 16,5 | 4,3 | 5,1 | 16,1 |
| 2ème mois de traitement | 18,7 | 4,5 | 4,9 | 19,2 |
| 3^{ème} mois de traitement | 20,2 | 5,2 | 4,8 | 21,3 |

Une nette différence est déjà visible pour le 1^{er} groupe dès le 1^{er} mois par rapport aux 2éme et 3éme groupes. L'efficacité est similaire à celle du 4^{ème} groupe (Minoxidil).

Cette étude confirme la synergie de l'association quercétine et acide laurique 1^{er} groupe par rapport à un éventuel effet additif :
- 1^{er} mois : 16,5 par rapport à 4,3+5,1(9,4) soit : 71 % d'augmentation
- 2^{ème} mois : 18,7 par rapport à 4,5+4,9 (9,4) soit : 98,9 % d'augmentation
- 3eme mois : 20,2 par rapport à 5,2 + 4,8 (10) soit : 102 % d'augmentation

Les compositions topiques de la nouvelle association contenant au moins de la quercétine avec au moins de l'acide laurique selon la présente invention sont illustrées par les exemples suivants :

### Référence Exemple 1 : Lotion capillaire A

| | |
|---|---|
| Quercétine | 100 mg |
| Acide laurique | 1 g |

| Excipients : | |
|---|---|
| Propylène glycol | 4 à 6 g |
| Alcool éthylique à 96 ° | 60 g |
| Eau déminéralisée stérile QSP | 100 g |

### Exemple 2 : Lotion capillaire B

| | |
|---|---|
| Quercétine | 200 mg |
| Acide laurique | 1 g |
| Caféine | 0,1 à 1g |

| Excipients : | |
|---|---|
| Propylène glycol | 4 à 6 g |
| Alcool éthylique à 96 ° | 50 à 80 g |
| Eau déminéralisée stérile QSP | 100 g |

### Référence Exemple 3 : Lotion capillaire C

| | |
|---|---|
| Quercétine | 200 mg |
| Acide laurique | 500 mg |

| Excipients : | |
|---|---|
| Tocophérylnicotinate | 0,3 à 0,6 g |
| Propylène glycol | 6 à 8 g |
| Alcool éthylique à 96 ° | 60 g |
| Eau déminéralisée stérile QSP | 100 g |

### Référence Exemple 4 : Lotion capillaire D

| | |
|---|---|
| Quercétine | 100 mg |
| Acide laurique | 200 mg |

| Excipients : | |
|---|---|
| Dimethicone copolyol (silicone Abil) | 100 à 200 mg |
| Tocophérylnicotinate | 0,1 à 0,3 g |
| Alcool éthylique à 96 ° | 40 à 60 g |
| Eau déminéralisée stérile QSP | 100 g |

### Référence Exemple 5 : Lotion capillaire E

| | |
|---|---|
| Quercétine | 200 mg |
| Acide laurique | 2 g |

| Excipients : | |
|---|---|
| Polyvinyl pyrrolidone | 0,1 à 0.3 g |
| Alcool éthylique à 96 ° | 70 à 80 g |
| Eau déminéralisée stérile QSP | 100 g |

### Référence Exemple 6 : Shampooing A

| | |
|---|---|
| Quercétine | 500 mg |
| Acide laurique | 300 mg |

| Excipients : | |
|---|---|
| Alcool cetyl-stearylique 9EO/2P 400 à 600 mg | 400 à 600 mg |
| Lauryl ether sulfate Na (2EO) 4 à 6 g | 4 à 6 g |
| Lauryl ether sulfate Na/Mg (3EO) 20 à 30 g | 20 à 30 g |
| Myristyl ether de suif (60 EO) 1 à 3 g | 1 à 3 g |
| Conservateurs ECOCERT | Sorbate de potassium 0,15g |
| | Benzoate de sodium 0,2g |
| Eau déminéralisée stérile QSP | 100g |

### Référence Exemple 7 : Shampooing B

| | |
|---|---|
| Quercétine | 250 mg |
| Acide laurique | 100 mg |

| Excipients : | |
|---|---|
| Alkylether sulfate de Na (2 EO) Empicol ESB 3R | 15 à 20 g |
| Cocoamphoglycinate 30% Ampholac XCO 30 | 10 à 15 g |
| Polymere de polyglycol polyamine Polyquart H81 | 0,5 à 1 g |
| Diethanolamide d'acide gras de copprah Oramide DL 200 | 0,3 à 0,8 g |
| Huile de ricin hydrogénée (40 EO) | 0,3 à 0,8 g |
| Acide lactique | QSP pH 6,5 |
| Chlorure de sodium | QSP viscosité 800cps |
| Conservateurs ECOCERT | Sorbate de potassium 0,15g Benzoate de sodium 0,2g |
| Eau déminéralisée stérile QSP | 100 g |

## Revendications

1. Composition topique dermatologique et/ou cosmétique comprenant l'association synergique d'au moins de la quercétine avec au moins de l'acide laurique dans un rapport compris entre 1 :3 et 1 :7 en poids.

2. Composition selon la revendication 1, comprenant de 0,01 à 0,5 % en poids de quercétine par rapport au poids total de la composition.

3. Composition selon l'une des revendications 1 à 2 comprenant de 0,05 à 2,5% en poids d'acide laurique par rapport au poids total de la composition.

4. Composition selon l'une des revendications 1 à 3, comprenant en outre de la caféine.

5. Composition selon la revendication 4, dans laquelle la caféine est présente entre 0,01 et 1 % en poids par rapport au poids total de la composition.

6. Composition selon l'une des revendications 1 à 5 dans laquelle ladite composition est sous forme de lotion et/ou de shampooing.

7. Composition selon l'une des revendications 1 à 6 pour son utilisation en cosmétique pour la stimulation de la croissance capillaire et comme anti- chute de cheveux.

8. Composition selon l'une des revendications 1 à 6 pour son utilisation comme médicament dans le traitement de l'alopécie.

9. Composition selon la revendication 8 pour son utilisation dans le traitement de l'alopécie androgénique, de l'alopécie aerata et de l'alopécie induite par la chimiothérapie.

## Patentansprüche

1. Dermatologische und/oder kosmetische topische Zusammensetzung, umfassend die synergistische Assoziation von mindestens Quercetin mit mindestens Laurinsäure in einem Gewichtsverhältnis zwischen 1:3 und 1:7.

2. Zusammensetzung nach Anspruch 1, umfassend von 0,01 bis 0,5 Gew.-% Quercetin im Verhältnis zum Gesamtgewicht der Zusammensetzung.

3. Zusammensetzung nach einem der Ansprüche 1 bis 2, umfassend von 0,05 bis 2,5 Gew.-% Laurinsäure im Verhältnis zum Gesamtgewicht der Zusammensetzung.

4. Zusammensetzung nach einem der Ansprüche 1 bis 3, außerdem umfassend Koffein.

5. Zusammensetzung nach Anspruch 4, wobei das Kaffein mit zwischen 0,01 und 1 Gew.-% im Verhältnis zum Gesamtgewicht der Zusammensetzung vorliegt.

6. Zusammensetzung nach einem der Ansprüche 1 bis 5, wobei die Zusammensetzung in der Form einer Lotion und/oder eines Shampoos ist.

7. Zusammensetzung nach einem der Ansprüche 1 bis 6, zu deren Verwendung in der Kosmetik zur Stimulation des Haarwuchses und als Mittel gegen Haarausfall.

8. Zusammensetzung nach einem der Ansprüche 1 bis 6, zu deren Verwendung als Medikament zur Behandlung von Alopezie.

9. Zusammensetzung nach Anspruch 8, zu deren Verwendung zur Behandlung von androgener Alopezie, Alopecia aearata und durch Chemotherapie induzierter Alopezie.

## Claims

1. A dermatological and/or cosmetic topical composition comprising the synergistic association of at least quercetin with at least lauric acid in a ratio ranging between 1:3 and 1:7 by weight.

2. The composition according to claim 1, comprising from 0.01% to 0.5% by weight of quercetin relative to the total weight of the composition.

3. The composition according to one of claims 1 to 2, comprising from 0.05% to 2.5% by weight of lauric acid relative to the total weight of the composition.

4. The composition according to one of claims 1 to 3, further comprising caffeine.

5. The composition according to claim 4, wherein caffeine represents between 0.01% and 1% by weight relative to the total weight of the composition.

6. The composition according to one of claims 1 to 5, wherein said composition is in lotion and/or shampoo form.

7. The composition according to one of claims 1 to 6, for use in cosmetics to stimulate hair growth and to prevent hair loss.

8. The composition according to one of claims 1 to 6, for use as a medicinal product for treating alopecia.

9. The composition according to claim 8, for use in treating androgenic alopecia, alopecia areata and chemotherapy-induced alopecia.
